# EUROPEAN PATENT SPECIFICATION

(11) **EP 0 448 923 B1**
(45) Date of publication and mention of the grant of the patent: **03.01.1996**
(21) Application number: 90890304.0
(22) Date of filing: 19.11.1990
(51) Int. Cl.: C12Q 1/04

(54) **Method and apparatus for detecting biological activities in a specimen**
Verfahren und Apparat zum Nachweis biologischer Aktivitäten in einer Probe
Méthode et dispositif pour la détection d'activités biologiques dans un échantillon

(30) Priority: 29.03.1990 US 501123
(43) Date of publication of application: 02.10.1991
(73) Proprietor: AVL PHOTRONICS CORPORATION, Roswell, GA 30077 (US)
(72) Inventor: Fraatz, Robert, Dr., Woodstock, Georgia 30188 (US); Joebstl, Ewald, Roswell, Georgia 30076 (US); Karpf, Hellfried, Dr., A-8010 Graz (AT)
(74) Representative: Krause, Walter, Dr. Dipl.-Ing.

(56) References cited:
- EP-A- 0 054 001
- EP-A- 0 104 463
- EP-A- 0 158 497
- EP-A- 0 171 158
- EP-A- 0 333 253
- WO-A-90/13663
- J.R. LAKOWICZ, "Principles of Fluorescence Spectroscopy", Chapter 9 ("Quenching of Fluorescence"), 1983, Plenum Press, New York/London; pp. 257-275/

## Description

The present invention relates to a method and to an apparatus for detecting biological activities in a specimen where the specimen and a culture medium are introduced into a sealable container and are exposed to conditions enabling metabolic processes to take place in the presence of microorganisms in the sample, the concentration of the initial substances being lowered and that of metabolic products being raised.

In many applications it is necessary to determine quickly whether a specimen is contaminated by microorganisms, such as bacteria, in particular in medical applications, in the pharmaceutical industry, food industry, or in environmental protection activities. The term "specimen" has a most comprehensive meaning here, including substances such as solid and liquid biological material (e.g. blood), food samples, such as frozen foods and preserves of canned foods, packaging material, clinical instruments and laboratory equipment, or samples taken from their surfaces, medical apparatus, first-aid and dressing material, soil and water samples, particularly samples of drinking water.

For a long time purely manual methods have been used in which the specimen to be assessed is placed in a culture bottle containing a liquid culture medium, and the growth of the culture is inspected only visually at given time intervals, and the type of presence of a microorganism is inferred from this observation by subculturing the liquid culture medium to a solid culture medium.

In addition, some technical procedures and devices are known, with which the biological activities in a sample are caused by microorganisms may be determined, and where the CO₂ produced by the metabolism of the microorganism, or rather, the change in CO₂ content, is employed as a measurement for determining the bological activity.

It is a known procedure, for example, to bottle the sample to be assessed together with a radioactively-labeled liquid culture medium and to test the atmosphere over the culture medium for radioactive gases, following which the presence of microorganisms in the sample may be determined.

Measuring systems of this type are described in US-A-3,076,679 and US-A-3,935,073, for example. Although such systems are quick and reliable, they have certain disadvantages, i.e. radioactive substances must be handled and samples must be repeatedly taken from the gas space above the culture medium for frequent monitoring. When the samples are removed from the gas space, the remaining samples to be monitored may easily be contaminated by the sample-taking element and measuring errors may occur.

In the European application 158 497, a system is disclosed in which the biological activity of the specimen is determined by means of infrared absorption. In this method, a specimen is introduced into a sealable vessel containing a liquid culture medium, and is tested for the presence of microorganisms. The vessel is subjected to specific conditions, i.e. certain temperatures are maintained over given periods of time, thus enhancing the metabolism of the microorganisms, during which process CO₂ is produced in the gas space above the culture medium by conversion of the carbon source. A sample is taken from the gas space and introduced into a measuring cell, and the CO₂ content is measured by infrared absorption. Again, the subsequent samples may be contaminated, and another drawback is that infrared absorption is a less-sensitive means of measuring than radioactive labeling.

In order to avoid the problem of cross-contamination, the European application 0 104 463 proposes a method and a device which are also based on the detection of CO₂ (produced by metabolic processes) by means of infrared absorption. In this method, no sample is taken, but infrared radiation is directly transmitted through the wall of the vessel into the gas space above the culture medium, and its absorption is determined. Due to this non-invasive measuring method, cross-contaminations are largely eliminated; the disadvantage of this method, however, is its lack of sensitivity compared to radiometric methods, as well as the fact that the measurement is distorted by other gas components absorbing radiation in the same frequency band as CO₂. A suitable example is the absorption bands of water vapor. The sample vessels employed must be permeable within a relatively narrow frequency range, which will only permit the use of specific materials for these vessels. An additional advantage is that the generation and filtering of the required infrared radiation is comparatively complex and expensive.

The EP-A-0 333 253 discloses a direct fluorescence detection method and describes a device and apparatus for monitoring changes in pH and CO₂ in a bacterial culture,utilizing optical absorption measurements of a pH indicator in a matrix. Although it is not as sensitive as the radiometric method, it does offer the advantage of being noninvasive and can be continuously monitored. The primary disadvantage is that since color changes are being measured, different optical systems must then be used when the indicator medium is changed, thereby limiting the apparatus to one or two sensors.

In the WO-A-90/13663 also a direct fluorescence detection method is disclosed. Further a device comprising pH- or CO₂-sensitive sensors, which are attached to a transparent wall of a container and being in direct contact with the specimen to be tested are disclosed. The methods and devices described in this document also require different optical systems when the indicator medium is changed.

In the EP-A-0 171 158 a method for detecting a specific group of microorganisms in a sample is disclosed. According to this method a medium containing L-tryptophan, an indicator of ferric ions and a fluorophor is used. Tryptophan-deaminase-positive microorganisms react with the L-tryptophan to produce indolpyruvic acid which reacts with the ferric ions to form a complex having an intense red brown coloration. A reduction in fluorescence of the culture shows the presence of tryptophan-deaminase-positive microorganisms. According to page 2, lines 21-29, the method disclosed in EP-A-0 171 158 is based on a fluorescence-quenching effect (see e.g., "Principles of Fluorescence Spectroskopy", J.R. LAKOWICZ, Plenum Press, 1983; pages 257-275), wherein a reaction between fluorophor and quencher occurs. That means the fluorophor disclosed in this document is not inert with respect to constituents of the sample.

It is therefore an object of the present invention to provide a method and an apparatus for detecting biological activities in a specimen, which have at least the same sensitivity as radiometric methods, and which offer the user a versatile, simple and inexpensive technique, providing him with information on the presence of microorganisms, while eliminating the danger of cross-contamination.

According to the present invention, the above object is achieved by:
introducing a sample of the specimen and a culture medium into a sealable transparent container containing at least one activable, inert fluorophor and at least one indicator component which changes its optical characteristics in response to changes in concentration of at least one substance in said container;
sealing said container;
exposing the specimen to conditions enabling metabolic activities to take place in the presence of microorganisms in the sample, wherein the concentration of at least one substance is altered during metabolic activities;
exciting said inert fluorophore to emit fluoroscent emission radiation;
modulating the fluorescent emission radiation of the inert fuorophore with the optical changes of the indicator to produce a modulated fluorescent signal; and
monitoring the signal over time for changes indicating the presence of microorganisms.
The inventive apparatus for detecting biological activities in a specimen comprises:
a sealable container including a transparent wall section;
a culture medium and a sample of the specimen therein sealed in said container;
means acting on the contents of said container for enabling metabolic activities which alters the concentration of at least one substance in said container;
at least one optical sensor attached to said transparent wall section, said optical sensor comprises an activable, inert fluorophore and an indicator component which changes its optical characteristics in response to changes in concentration of at least one substance in said container caused by metabolic activities, wherein the fluorescent emission radiation of the inert fluorophore is modulated by the optical changes of the indicator component to produce a modulated fluorescent signal;
excitation means for illuminating said at least one optical sensor through said transparent wall section of said container to excite said inert fluorophore of said optical sensor to emit the respective fluorescent signals;
detecting means optically coupled through said transparent wall section of said container to said at least one optical sensor for detecting the changing fluorescent signals.

The optical sensors or optodes which rely on the principle of fluorescence attenuation or enhancement, which may be inexpensively mass produced and which are in direct contact with the substance to be assessed and attached to a transparent surface of the container (internal filtered effect), permit continuous monitoring in closed systems, where new optodes are used for each sample, such that cross-contaminations are eliminated in the most simple variation, for example, a single sensor may suffice to determine the presence of microorganisms by way of the changes in substance concentrations relative to the initial concentrations.

With respect to the aforementioned disadvantage of measuring color changes and the requirement for different optical systems when an indicator medium is changed, another object of the present invention is to provide a sensor which allows the monitoring of many optical changes while utilizing the same optical system.

According to the present invention, the immediately-foregoing object is achieved by utilizing an activable inert fluorophore in the optical sensor and measuring the modulation of the fluorescent output caused by optical changes in the indicator component. It also provides for very sensitive continuously monitoring a noninvasive system.

The present invention provides that concentrations be measured of at least one substance from the group of CO₂, O₂, H⁺ (pH), NH₄ ⁺, H₂ and metal ions, the indicator component of the optical sensor responding to a change in substance concentrations by changing its optical characteristics such that a change in fluorescence intensitiy from an inert fluorescing component of the sensor is measured.

In particular, the invention provides, for example, for the testing of blood samples, that the CO₂ concentration be measured continuously and the detection of microorganisms be determined upon a rise of the CO₂ concentration. The invention further provides for using any combination of sensors to identify the microorganisms completely or partially.

In accordance with another variation, the invention will also permit continuous measuring of the O₂ concentration and determination of the changes in NH₄ and pH. The advantage of this variation is that the significant changes in the O₂, NH₄, concentration and pH may be detected earlier than the detection of changes of CO₂ alone.

It is also provided that a culture medium containing a carbon compound be introduced into a sealable container, and that the sensor be added to the culture medium, which responds to the changes in metabolic substances content by a change in its fluorescence behaviour, and that a blood sample be introduced into the container, which may be subject to metabolic processes in the presence of microorganisms, during which metabolic processes occur, and that the content of the container be exposed to excitation radiation and the radiation emitted by the fluorescent component of the sensor be measured, a change in fluorescence behavior indicating the presence of microorganisms. For example, indicator capsules as disclosed in the DE-A-2 360 384 may be added, or rather, microcapsules containing sensors, whose walls are made from polymerized hydrophilic monomers.

According to the invention, at least two of said optical sensors may be combined to form a bisensor for indicating concentration changes of O₂ and pH, or at least three of said optical sensors may be combined to form a trisensor for indicating concentration changes of CO₂, O₂ and pH.

At this point, a description shall be provided with respect to the concepts, types of structure on a system and component basis, technology, principles of the invention and model systems for particular materials, all included in what is referred to as Fluorescence Intensity Modulation Sensor Technology (FIMST).

The present invention is based on certain fundamental concepts including the desire to use an inert fluorescent material in combination with an aqueous suspension of an indicator to provide significant benefits due to:
1. Selection of a high quantum efficiency fluorophore;
2. Selection of fluorophore that has an absorbance match to an inexpensive and powerful excitation source;
3. Selection of an indicator that is most sensitive to the region of interest;
4. Selection of an indicator with a strong absorbance at either, or both, the excitation or emission wavelength of the fluorophore; and
5. The potential to use the same optical system (source and detector) for sensors detecting different analytes.

The structure of embodiments of the invention may utilize various components and variations including:
1. Membrane (e.g. gas permeable silicone)
   a) single membrane containing both the inert fluorophore and the indicator component, or
   b) a multi-layer structure with the inert fluorophore and the indicator component in separate layers;
2. Fluorescent material, fluorophore
   a) contained in the silicone, or
   b) contained in an aqueous emulsion;
3. Indicator
   a) contained in the aqueous emulsion,
   b) contained in the sample solution, or
   c) contained in the sensor matrix;
4. Source
   a) light emitting diodes (LEDs) including
      1. blue 456 nm peak or 470 nm peak,
      2. green 560 nm peak, 550--580 nm half intensity,
      3. yellow 590 nm peak, 570---610 nm half intensity,
      4. high intensity red (red/orange) 645 nm peak, 615--660 nm half intensity and
      5. red 665 nm peak, 650--670 nm half intensity; and
   b) other lamps such as tungsten, quartz halogen and neon; and
5. Detector
   a) silicon photodiodes,
   b) PIN silicon diodes,
   c) GaAsP photodiodes, and
   d) other photodetectors including photovoltaics, photoresistive devices and photoconductive devices.

The method diclosed herein has general applicability for the determination of any substance for which a colorimetric method and a permeable membrane can be prepared. Various principles may be used in selecting a sensor system and model sensor systems are discussed hereinbelow.

According to the principles of the invention, current optical sensors using fluorescence or absorbance-based materials have inherent limitations. The absorbance and fluorescence-based systems must use different optical systems (light source, filters and detectors) for each sensor system, depending on the optical change of the indicator medium. This is due to the necessity of providing and measuring different wavelengths of light for each different optical system.

The disadvantage for direct fluorescence detection of the metabolic substances of microorganisms is that the source and detectors for use in fluorescence are expensive and of limited availability. In addition, direct fluorescence systems have not yet been described for some tests of interest (e.g., ammonia).

A system constructed in accordance with the present invention has a sensor and an optical system that provides for the determination of a fluorescent intensity from the sensor. The optical unit has a light source that provides light of the appropriate wavelengths to excite the fluorescent material in the sensor. The light source may be filtered to provide light only at the wavelengths appropriate to the fluorophore. In the most simple case, the source is a light-emitting diode that provides transmission only at the wavelengths needed for excitation.

The detector can be any device that will provide an electronic signal proportional to the light intensity. The preferred embodiment uses a silicon photodiode with a filter to select only the fluorescent wavelength. In a preferred variation, the source and detector are held in a mount that positions the light from the lamp to illuminate the sensor directly above the photodetector. Appropriate optical filters are used to optimize the signal change associated with the optical change in the indicator medium.

The sensor is composed of two parts, an inert fluorescent material which provides the fundamental optical signal and, the indicator material or indicator component. In practical applications, the sensor is placed and attached to a transparent surface inside a sealable container that can be filled with liquid media that supports the growth of microorganisms.

The fluorescent material is selected to provide an optical signal matched with the source and detector. The quantum efficiency of the fluorescent material will, to an extent, determine the detection range of the sensor system. The fluorescent material may be mixed in the sensor with the indicator material, incorporated as a part of the sensor matrix, applied as a coating to the back of the sensor or in any manner in which the light from the source will pass through the fluorescent material before or after passing through the indicator material. The indicator material is selected to provide an optical change in response to the analyte at the concentrations expected. The sensitivity of the sensor system is determined by the amount which the color changes of the indicator interacts with (modulates) the excitation and/or emission of the fluorescent signal. In use, the sensor system provides a constant signal when the concentration of the analyte remains constant. When the analyte concentration changes, the indicator responds by exhibiting a change in optical properties (e.g., color intensity). This change acts as an optical filter to change the amount of light exciting or emitted from the inert fluorophore. This change in light is detected as an amplitude change (modulation) by the appropriately-filtered photodetector.

A number of different configurations are possible in this sensor system including having separate layers of silicone membrane for the fluorescent material and the indicator component. In a preferred variation, the optode is covered with a dye-impregnated silicone to maintain optical isolation for the system. Examples of the construction of the sensor are presented in Tables I, II and III. An example of using this optical system for the detection of carbon dioxide and pH are presented. In these model systems, the optical unit remains the same even though the indicator component changes.

Several model system examples will now be provided.

The optical system may comprise a yellow LED source and an output of 63 mcd at a peak wavelength of 590 nm. The detector is a silicon photodiode with a peak sensitivity at 720 nm and 0.37 W/A sensitivity at 660 nm.

A model system for the detection of CO₂ is disclosed below. The fluorescent material carboxynaphthofluorescein is suspended in a matrix of carbon dioxide-permeable silicone polymer. Included in this matrix is an aqueous suspension of Bromthymol Blue indicator. The fluorescent material is excited by using the yellow LED with a peak emission of 590 nm and a bandwidth of 40 nm. This corresponds well to the peak absorbance for the fluorescent material carboxynaphthofluorescein at 598 nm. The emission of the fluorescent material is maximum at 660 nm. The sensor is in equilibrium with the carbon dioxide present in the media and this results in the aqueous emulsion of the sensor having a pH of greater than 7.6. If bacteria are present in the sample, the CO₂ produced thereby diffuses through the silicone membrane into the aqueous emulsion and reduces the pH of the emulsion. As the pH is reduced, more of the Bromthymol Blue indicator is converted to the acid form and the color of the emulsion changes from blue to yellow (e.g., peak absorbance changes from 617 nm to 470 nm and the yellow light is not strongly filtered out).

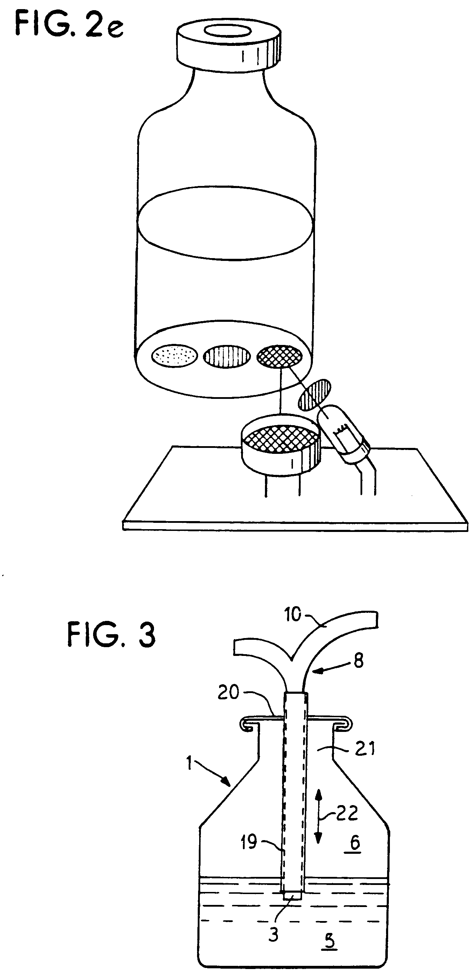

When the indicator is in the base form (blue) it filters out the excitation light and no fluorescent signal is detected. As the pH and the emulsion decreases and more of the base form is converted to the yellow acid form, and more yellow light is allowed to reach the fluorescent material and an optical signal is detected. The light intensity is found to increase with an increase in CO₂ concentration. Experimentally, however, the operational amplifier inverts the signal and in Fig. 3 the voltage signal is decreased with an increase in carbon dioxide.

A model sensor system for pH measurement uses the same Bromthymol Blue indicator as does the carbon dioxide sensor. However, instead of having the gas-permeable silicone membrane, this sensor has the indicator immobilized on a support and protected by a hydrogen ion-permeable membrane. As the pH of the media changes, the ratio of Bromthymol Blue in the acid to base form changes in a manner proportional to the hydrogen ion concentration. This change in color provides a change in the fluorescent signal that is also proportional to the hydrogen ion concentration. This value can be used to determine the pH, as pH = -log (hydrogen ion concentration). The range of this sensor is limited to the range in color change of Bromthymol Blue, 6.2 to 7.6 pH. This range, however, covers the physiological pH range. Should an increase in the range be required, there are other sensor systems listed herein that cover the range of pH from 4.7 to 8.0.

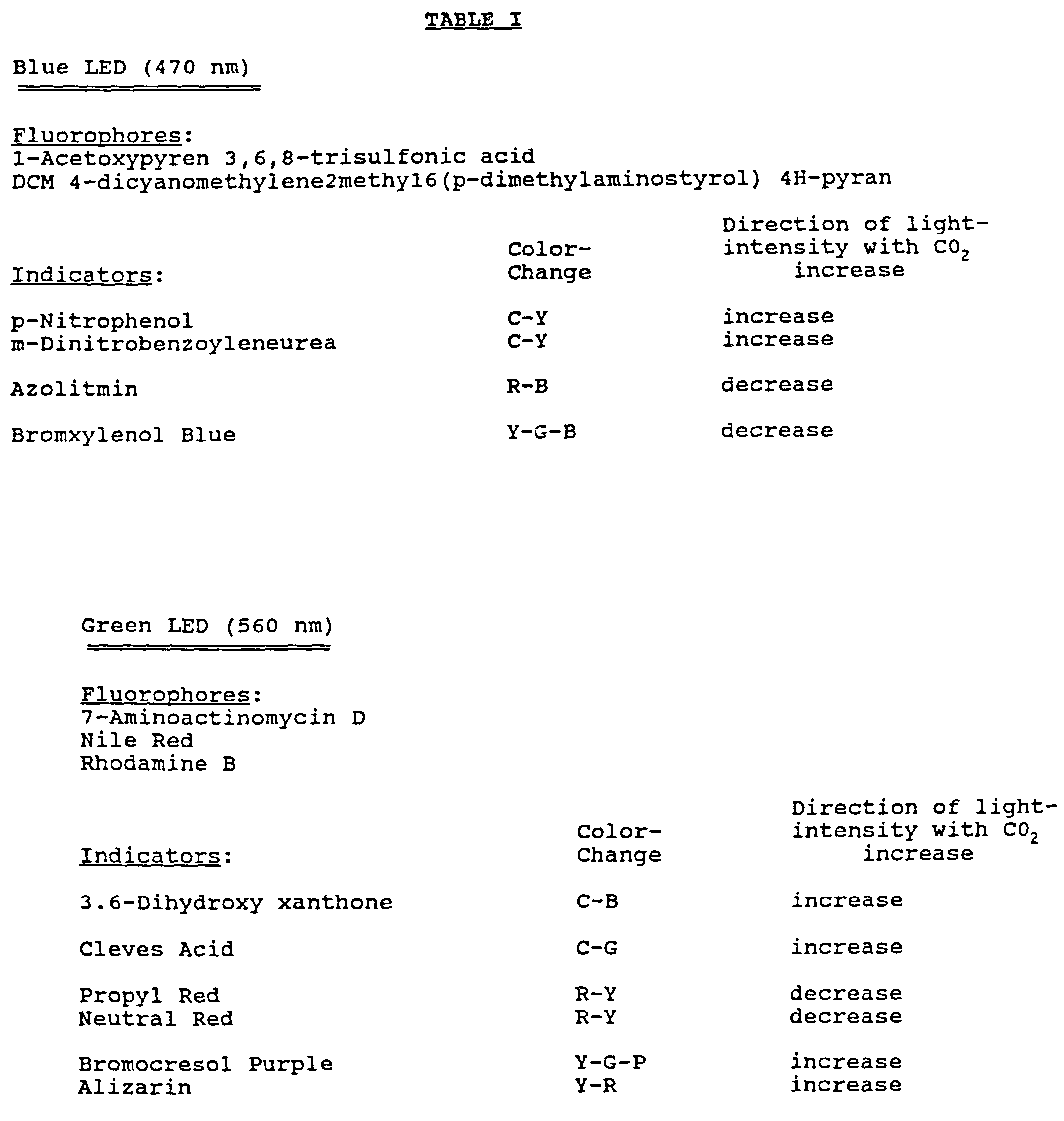

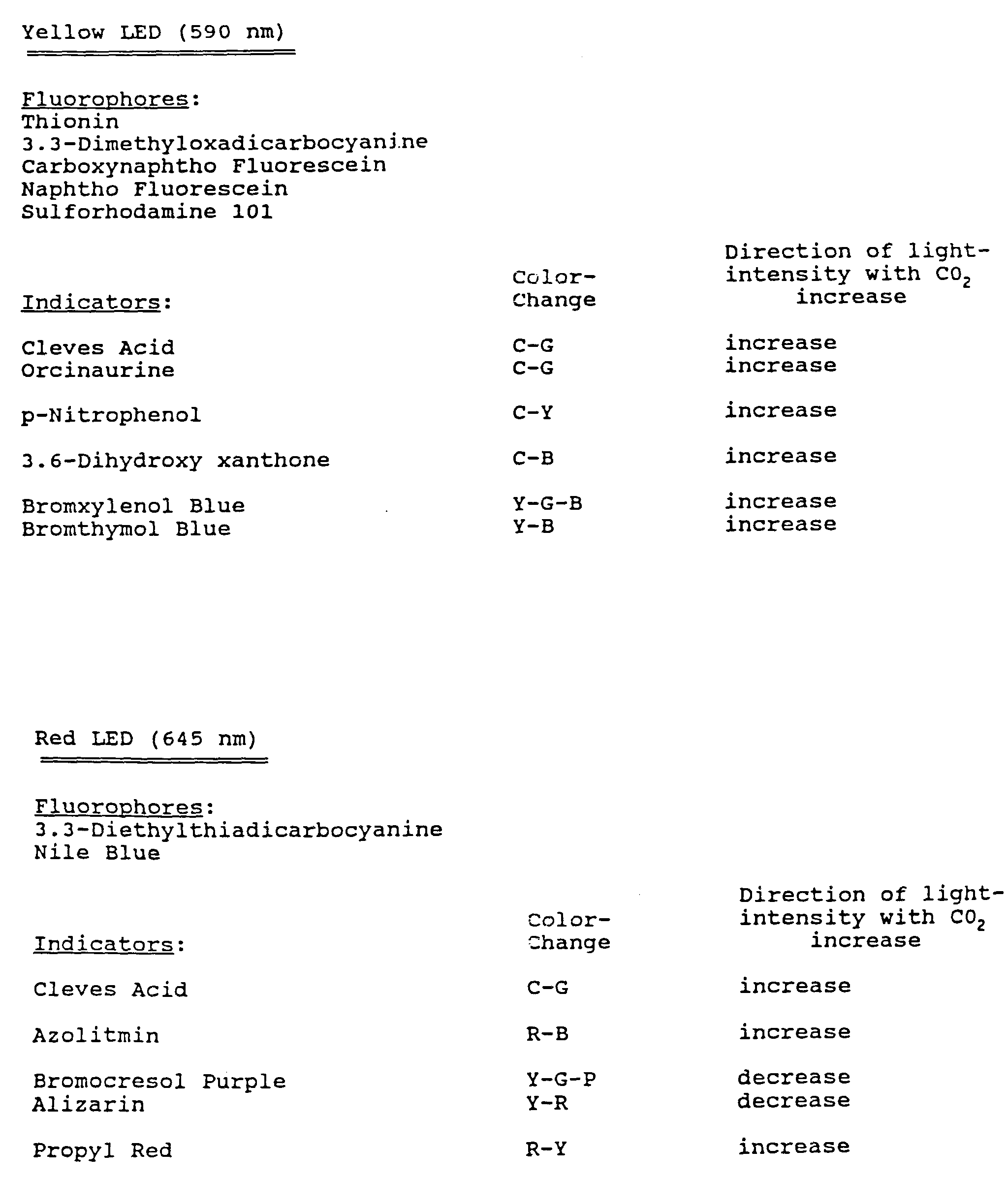

**TABLE II**

| **FLUORESCENT DYES AND PIGMENTS** | | | |
|---|---|---|---|
| Abbr. | Dye | Absorbance Max. nm | Fluorescent Max. nm |
| HPTS | 1Hydroxypyren368trisulfonicacid | 460 | 515 |
| APTS | 1Acetoxypyren368trisulfonicacid | 460 | 515 |
| RHO-123 | Rhodamine 123 | 505 | 534 |
| RHO-110 | Rhodamine 110 | 510 | 535 |
| EO | EOSIN | 518 | 550 |
| 7-AAD | 7-Aminoactinomycin D | 523 | 647 |
| SFRHO-G | Sulforhodamine G | 529 | |
| RHO-6G | Rhodamine 6G Perchlorate | 530 | 556 |
| RHO-6G | Rhodamine 6G Perchlorate | 530 | 590 |
| EVA | Evans Blue | 550 | 610 |
| NIRE | Nile Red Phenoxazon 9 | 551 | 636 |
| RHO-B | Rhodamine B | 552 | 580 |
| RHO-B | Rhodamine B | 554 | 627 |
| PYRO | Pyronin B | 555 | 599 |
| SFRHO-B | Sulforhodamine B | 556 | 575 |
| DODC | 3.3-Dimethyloxadicarbocyanine | 582 | 660 |
| SFRHO-101 | Sulforhodamine 101 | 586 | 607 |
| NAPH-FLU | Naphtho Fluorescein | 594 | 663 |
| CARB-FLU | Carboxynaphtho Fluorescein | 598 | 660 |
| TION | Thionin | 599 | 850 |
| NIBL | Nile Blue A Perchlorate | 628 | 690 |
| DTDC | 3.3- Diethylthiadicarbocyanine | 653 | 760 |
| DOTCI | Methyl-DOTCI | 682 | 718 |
| IR | IR 144 | 750 | 848 |

**TABLE III**

| ABBR. | INDICATORS | ACID FORM | COLOR CHANGE | BASE FORM |
|---|---|---|---|---|
| PYR | Propyl Red | 4.7 | R-Y | 6.6 |
| NIPH | p-Nitrophenol | 4.7 | C-Y | 7.9 |
| ACOL | Azolitmin | 5.0 | R-B | 8.0 |
| BROMCRE | Bromocresol Purple | 5.2 | Y-G-P | 6.8 |
| CHLORO | Chlorophenol Red | 5.4 | Y-R | 6.8 |
| DX | 3.6-Dihydroxy xanthone | 5.4 | C-B | 7.6 |
| ALI | Alizarin | 5.6 | Y-R | 7.2 |
| BROXBL | Bromxylenol Blue | 5.7 | Y-G-B | 7.5 |
| DPD | 3.6-Dihydroxy phthalic dini | 5.8 | B-G | 8.2 |
| NITEU | m-Dinitrobenzoyleneurea | 6.0 | C-Y | 7.8 |
| BROMBL | Bromthymol Blue | 6.2 | Y-B | 7.6 |
| AU | Aurin (Aosolic acid) | 6.3 | Y-P | 6.9 |
| PHENRE | Phenol Red | 6.4 | Y-O-R | 8.0 |
| CLEV | Cleves Acid | 6.5 | C-G | 7.5 |
| ORC | Orcinaurine | 6.5 | C-G | 8.0 |
| RES | Resolic acid | 6.8 | Y-R | 8.0 |
| NEURE | Neutral Red | 6.8 | R-Y | 8.0 |

| pH-Indicators | | | | |
|---|---|---|---|---|
| B-Blue | -500 nm | | | |
| | | | | |
| G-green | 510-590 nm | | | |
| Y-yellow | 590-620 nm | | | |
| O-orange | 620-640 nm | | | |
| R-red | 640-700 nm | | | |
| P-purple | 700-750 nm | | | |
| C-colorless | | | | |

As indicated above, according to invention, optodes are provided for selecting detection of at least one substance from the group of CO₂, O₂, H₂, H⁺ (pH), NH⁺ ₄ and metal ions that are present during the metabolic process, as initial, intermediate or the final products.

The excitation and detection assembly may comprise a LED light source and a photodiode detector as well as a two-armed optical waveguide transmitting excitation radiation to the optode or optodes at carrying of the optical signal to the detector.

In a preferred variation of the invention, the optodes are combined to form a multilayer sensor.

In a simple structure of the invention, the optodes or optical sensors are attached to the inside of the wall of a transparent container and are connected to the evaluation unit via the excitation and detection assembly that may be placed flush against the outside of the wall of the container. The optodes, which may be mass produced inexpensively, are attached directly or with an adhesive to the inner surface of the wall of the sample container, which is then filled with the culture medium, sealed and stored. After the addition of the specimen, for example, a blood sample, the container is thermostat-controlled for the time required for the growth of the culture, and is shaken if necessary, whereupon the concentration of the substance subject to chemical reaction by the metabolic processes is measured, for example, with a suitable optically-filtered photodiode in close proximity to the optode.

According to the invention, it is possible to place at least the optodes in the gas space of the at least partially transparent container above the culture medium mixed with the sample, and to use these optodes for measuring the concentration of at least one gaseous metabolite.

It is provided in accordance with the further variation of the invention that the optodes be located on a transparent stopper used for sealing the container.

The method will also permit, however, to place the optodes in a portion of the container covered by the culture medium mixed with the sample, possibly at the bottom of the container, and to use the optodes for measuring the concentration of at least one substance in the culture medium. With this arrangement, the metabolite is measured immediately at the place where it is produced, which will permit more rapid assessment as to whether a culture is positive or negative.

Other objects, features and advantages of the invention, its organization, construction and operation will be best understood from the following detailed description, taken in conjunction with the accompanying drawings, on which:
- Fig. 1: is a schematic representation of a device for practicing the invention;
- Figs. 2a, 2b, 2c, 2d, 2e, 3 and 4: are similar views of variations of the device of Fig. 1;
- Fig. 5: is a schematic representation of a variation for automatic measuring of several containers at the same time;
- Figs. 6 and 7: are schematic representations of variations of the structure of Fig. 5;
- Figs. 8, 9 and 10: are graphic illustrations of curves of measured concentrations; and

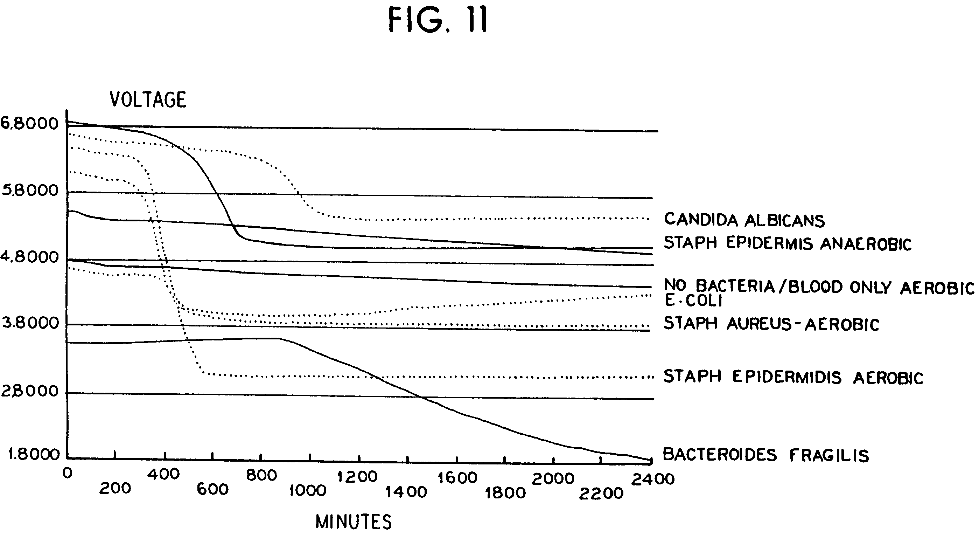

- Fig. 11: is a graphic illustration of measured curves using the sensor of the present invention to detect the growth of a variety of bacteria commonly found in cases of bacteremia.

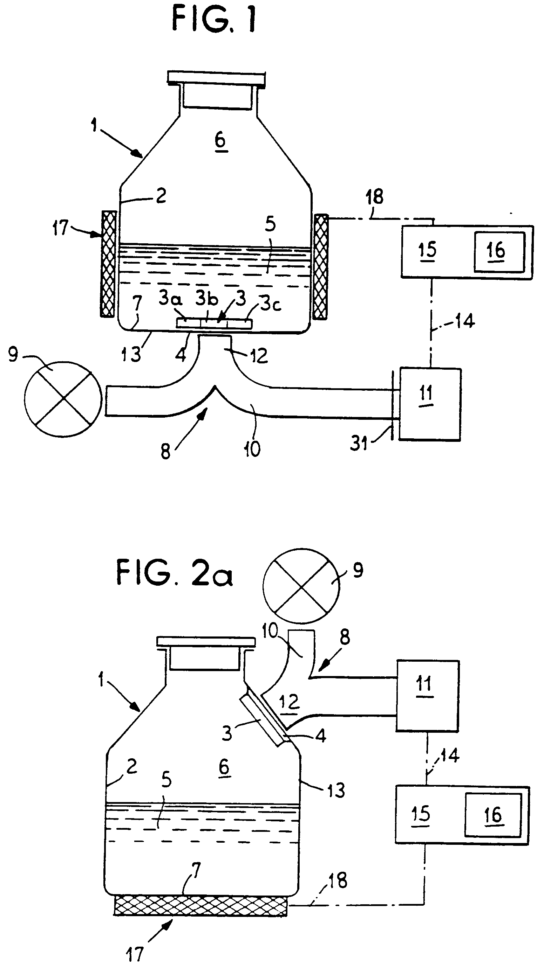

The device of Fig. 1 for detecting biological activities in a sample comprises a sealable, optically-transparent container 1 with an optode or optical sensor 3 attached to the inner surface 2 of its wall and bonded by a transparent adhesive layer 4.

Instead of a single optode 3 for a substance to be assessed, two or more optodes, 3a, 3b and 3c may be combined into a multilayer sensor, which will permit simultaneous detection of the changes in O₂ and CO₂ concentrations and in pH, for example. The individual optodes 3a to 3c or their indicator media may be stacked in layers one above the other, or they may be imbedded in a polymer membrane in homogenous distribution. The combination of a C₂ and an O₂ optode into a sensor is described in the European application 0 105 870, for example.

Instead of the optode 3 in the variations discussed below, optodes may be provided for measuring O₂, CO₂, H⁺ (pH), NH₄ ⁺ and H₂, or rather, a specific combination of these optodes in accordance with the particular requirements.

The container 1 contains the culture medium 5 with one carbon compound (glucose) , for example, which is converted by metabolic processes of microorganisms in the sample and to metabolic product, for example CO₂, during which processes O₂ is being consumed and the pH is subject to change. As a consequence, there are changes in the concentration of the metabolic product and the initial substances and the gas space 6 above the culture medium 5 and in the culture medium itself, which are detected by way of the optodes 3a, 3b and 3c placed at the bottom 7 of the container 1 in Fig. 1. The excitation and detection assembly 8 comprises a light source 9, a detector 11 and a two-armed light waveguide 10, one of whose arms is coupled to the light source 9 and the other of which is coupled to the detector 11. The end 12 of the light waveguide is placed flush against the exterior 13 of the wall of the container, transmitting excitation radiation towards the optodes 3a, 3b and 3c through the transparent wall of the container, while receiving the optical signal, e.g., the fluorescence radiation emitted by the optodes.

The use of a suitable filter 31, for example, a filter disc, at front of the detector 11 will ensure that the signals are assigned to their corresponding optodes 3a, 3b, 3c.

By way of a line 14, the detector signals are transmitted to an evaluation unit 15 in which the change over time, e.g., of the CO₂ content is determined and the status of the sample is indicated via a display 16.

The conditions in the container necessary for the metabolic processes are maintained by way of the unit 17, which is mainly responsible for proper temperature control of the sample, and is connected with the evaluation unit 15 via a control lead 18.

Instead of being a 17, an air heating element may be used for sample temperature control of the variation of Fig. 1 and all subsequent variations.

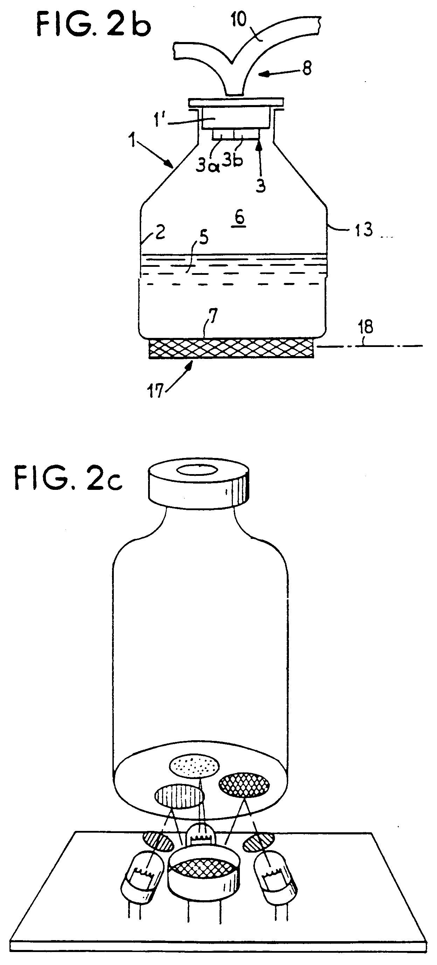

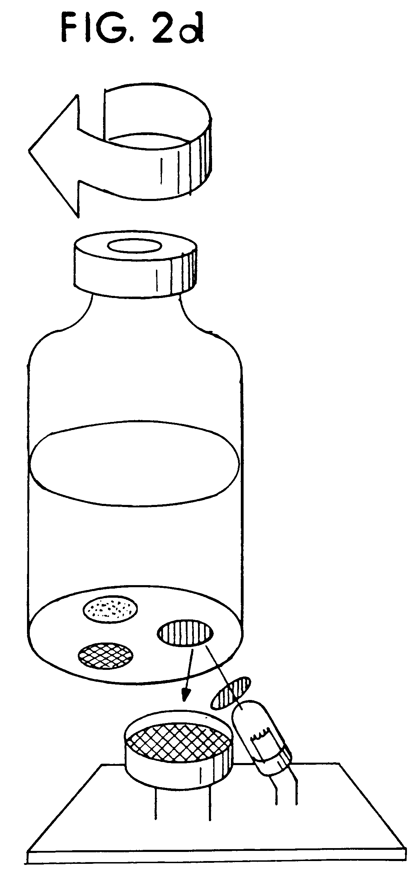

The structure illustrated in Fig. 2a differs from that of Fig. 1 only by the fact that the optode 3 is located in the gas space 6 of the container 1 and that the metabolites may be measured only. In this instance, temperature control is performed via the bottom 7 of the container 1. In a structure according to Fig. 2b, the optode 3, or the optodes 3a, 3b, may be attached to a stopper 1' sealing the container 1. The light waveguide 10 may go either through this stopper 1' or it may be placed on the exterior of the transparent stopper 1', as is shown in Fig. 2b. Figs. 2c, 2d and 2e illustrate other modifications of Fig. 1 which utilize LEDs and photodiode detectors even though each optode has a different chemistry and produces a different optical change (e.g., color). The sensor's optical system is being used because of the fluorescence is being measured.

In another structure as presented in Fig. 3, the optode 3 is attached to the tip of a probe 19 receiving the end of the two-armed light waveguide 10. The probe 19 is introduced into the container 1 through the opening 21 sealed by a septum 20, and may be axially shifted along in the direction of the arrow 22, permitting measurements to be taken both in the gas space 6 and in the culture medium 5.

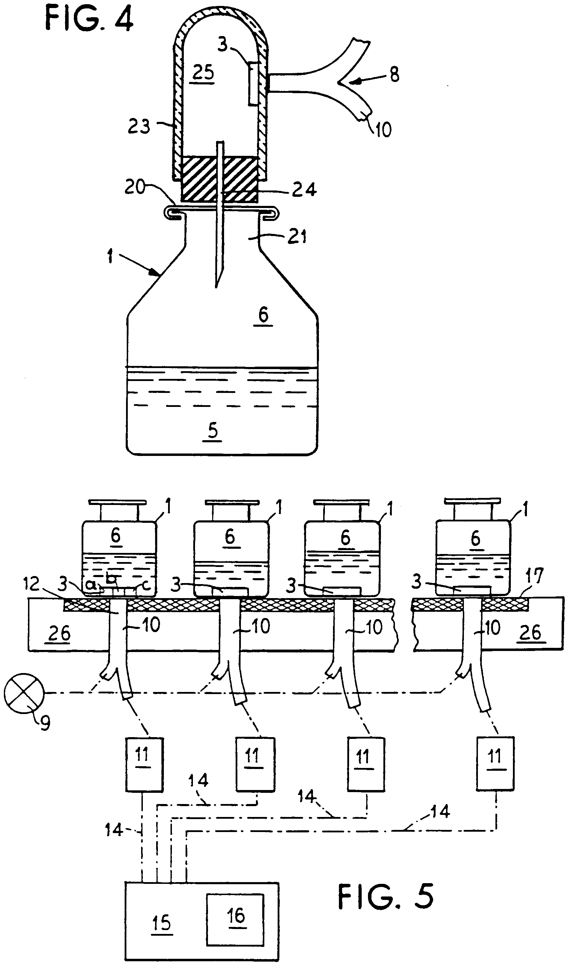

In the structure illustrated in Fig. 4, the optode 3, which is used, for example, for measuring O₂ and CO₂, is not located in the container 1, but is placed in a sampling vessel 23. In order to introduce the sample into the culture bottle, the septum 20 of the container 1 is punctured with the hollow needle 24 of the sampling vessel 23, permitting the sample to enter the culture medium. Via the hollow needle 24 a gas exchange will take place between the gas space 6 and the interior 25 of the sampling vessel 23 such that the change in the concentration of CO₂ and O₂ may be determined with the use of the excitation and detection assembly 8 which is symbolically indicated by the light waveguide 10.

A particularly favorable structure is illustrated in Fig. 5 comprising a device 26 configured as a temperature-controlled supporting rack, which will hold several containers 1 at the same time. The containers are inserted in labeled positions and are arranged in several rows, permitting temperature control and continuous monitoring of up to 600 containers simultaneously. Each container is assigned a two-armed light waveguide 10 located in the supporting rack 26 and providing the optodes 3a to 3c at the bottom of each container 1 with excitation radiation. The corresponding optical signals are delivered to the individual detectors 11 connected to the evaluation unit 15 via lines 14. The individual readings delivered to the evaluation unit 15 are accompained by suitable position identification signals such that the individual values may be directly assigned to the corresponding sample.

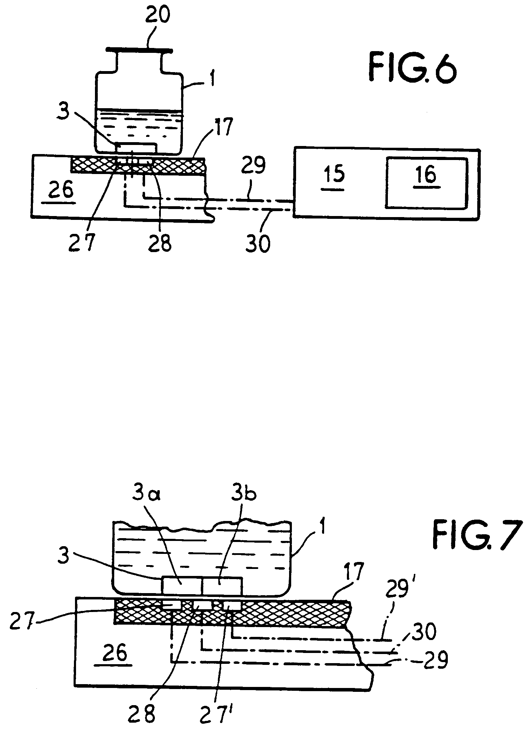

A structure as presented in Fig. 6 provides that each individual container 1 be connected with a LED 27 located in the supporting rack 20 and with a photodiode 28, possibly in conjunction with photo elements. In this manner, a most compact device is obtained which is characterized by the total absence of moveable parts.

Fig. 7 illustates a structure according to Fig. 6, which contains two optodes, 3a and 3b, combined into a sensor (e.g., a bisensor), for simultaneous measurement of O₂ concentration and pH. The optodes are excited via different LEDs 27 and 27' whose emission radiation is received by a common photodiode 28. The corresponding electrical leads 29, 29', 30 lead to the evaluation unit not shown here. By means of known optical or electronic equipment, the signals of the two optodes may be separated. Other variations with only one LED for excitation and several photodiodes for signal detection are within the scope of this invention.

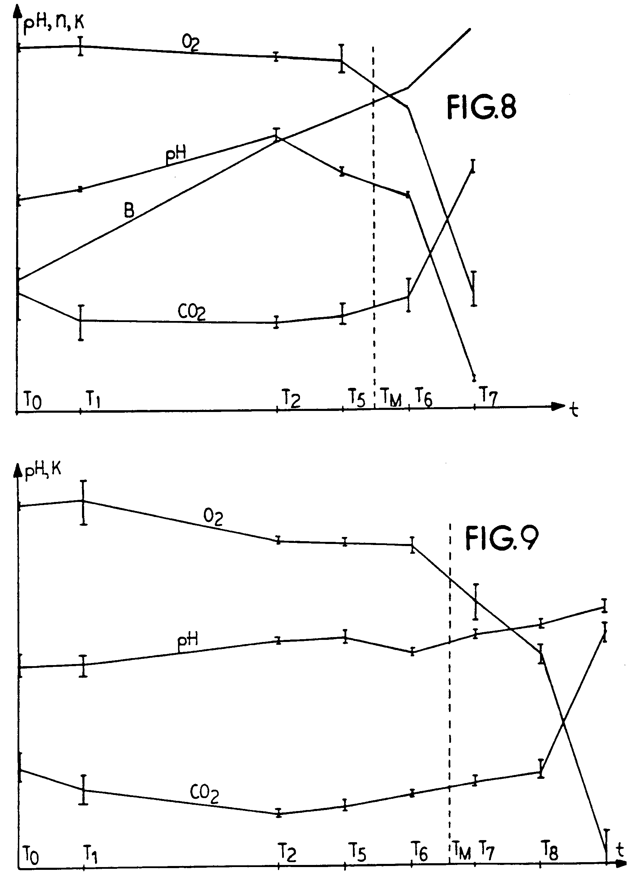

In the graphic illustrations of Figs. 8 and 9, the time t, or rather, the individual points in time T₀ to T₈ are plotted on the abscissa, while the pH value, the concentration K (or the partial pressure of O₂ and CO₂) and the number of bacteria or organisms per unit volume (logarithmic scale) are plotted on the ordinate.

Fig. 8 illustrated the change over time of the parameters O₂, CO₂ and pH using a sample containing Staphylococcus areus. Between the times T₅ and T₆ the concentration of CO₂ is charaterized by a significant increase indicating a positive sample, and about the same time the O₂ and pH levels decrease.

As opposed to Fig. 8, the pH value in Fig. 9 remains largely unchanged, whereas the O₂ decrease occurs significantly sooner than the CO₂ increase. In this instance, a sample with Psaudomonas aeruginosa was used.

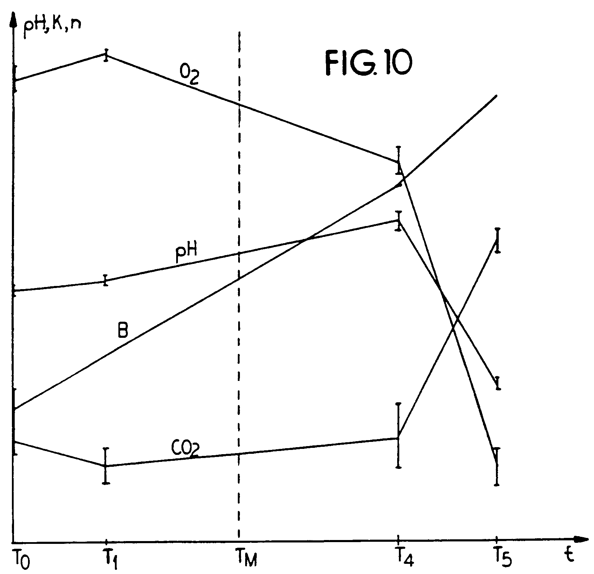

The values provided in Fig. 10 are taken from a sample containing enterobacteria (E.Coli). All three parameters change and once again the O₂ decrease was noticed prior to the changing of the other parameters.

The method and device described herein are well-suited for detecting biological activities and samples, for example, of microorganisms in blood, (bacteriemia, septicemia or pyemia).

The continuous, noninvasive monitoring of specimens helps to obtain fully automated incubation and measuring processes for a large number of samples. Positive cultures are quickly identified and erroneous negative findings are avoided.

## Claims

1. A method for detecting biological activities in a specimen, comprising:
introducing a sample of the specimen and a culture medium into a sealable transparent container containing at least one activable, inert fluorophor and at least one indicator component which changes its optical characteristics in response to changes in concentration of at least one substance in said container;
sealing said container;
exposing the specimen to conditions enabling metabolic activities to take place in the presence of microorganisms in the sample, wherein the concentration of at least one substance is altered during metabolic activities;
exciting said inert fluorophore to emit fluoroscent emission radiation;
modulating the fluorescent emission radiation of the inert fluorophore with the optical changes of the indicator to produce a modulated fluorescent signal; and
monitoring the signal over time for changes indicating the presence of microorganisms.

2. A method according to claim 1, wherein said sealable transparent container is provided with an optical sensor which comprises an inert fluorophore and a CO₂-sensitive color indicator and wherein the fluorescent signal of the fluorophor is modulated with the CO₂-sensitive color indicator in response to the increasing CO₂ concentration.

3. A method according to claim 1, wherein the intensity of the fluorescent emission radiation emitted by the inert fluorophore is modulated by the at least one indicator component in response to its optical characteristics.

4. A method according to claim 1, wherein the radiation, prior to exciting the inert fluorophore is modulated by the at least one indicator component in response to its optical characteristics.

5. Apparatus for detecting biological activities in a specimen, comprising:
a sealable container including a transparent wall section;
a culture medium and a sample of the specimen therein sealed in said container;
means acting on the contents of said container for enabling metabolic activities which alters the concentration of at least one substance in said container;
at least one optical sensor attached to said transparent wall section, said optical sensor comprises an activable, inert fluorophore and an indicator component which changes its optical characteristics in response to changes in concentration of at least one substance in said container caused by metabolic activities, wherein the fluorescent emission radiation of the inert fluorophore is modulated by the optical changes of the indicator component to produce a modulated fluorescent signal;
excitation means for illuminating said at least one optical sensor through said transparent wall section of said container to excite said inert fluorophore of said optical sensor to emit the respective fluorescent signals;
detecting means optically coupled through said transparent wall section of said container to said at least one optical sensor for detecting the changing fluorescent signals.

6. An apparatus according to claim 5, wherein:
said optical sensor is responsive to indicate changes in concentration of one substance selected from the group consisting of CO₂, O₂, H⁺ (pH), NH₄ ⁺, H₂ and metal ions.

7. An apparatus according to claim 5, wherein:
at least two of said optical sensors are combined to form a bisensor for indicating concentration changes of O₂ and pH.

8. An apparatus according to claim 5, wherein at least three of said optical sensors are combined to form a trisensor for indicating concentration changes of CO₂, O₂ and pH.

9. An apparatus according to claim 5, wherein one of said optical sensors is a CO₂ sensor and comprises:
a CO₂-permeable silicone matrix;
carboxynaphthofluorescein fluorescent material suspended in said matrix; and
a Bromthymol Blue aqueous emulsion as an indicator in said matrix.

10. An apparatus according to claim 5, wherein said inert fluorophore comprises a material selected from the group consisting of 1-acetoxypyren, 3,6,8-trisulfonic acid and DCM 4-dicyanomethylene-2-methyl-6 (p-dimethylaminostyrol) 4H-pyran and said indicator component comprises a material selected from the group consisting of p-Nitrophenol, m-Dinitrobenzoyleneurea, Azolitmin and Bromxylenol Blue.

11. An apparatus according to claim 5, wherein said inert fluorophore comprises a material selected from the group consisting of Thionin, 3.3-Dimethyloxadicarbocyanine, Carboxynaphthofluorescein and Sulforhodamine 101 and said indicator component comprises a material selected from the group consisting of Cleves Acid, Orcinaurine, p-Nitrophenol, 3.6-Dihydroxy xanthone, Bromxylenol Blue and Bromthymol Blue.

12. An apparatus according to claim 5, wherein said inert fluorophore comprises a material selected from the group consisting of 3.3-Diethylthiadicarbocyanine and Nile Blue, and said indicator component comprises a material selected from the group consisting of Cleves Acid, Azolitmin, Bromocresol Purple, Alizarin and Propyl Red.

13. An apparatus according to claim 5, wherein said inert fluorophore comprises a material selected from the group consisting of 1-Hydroxypyren-3,6,8-trisulfonic acid, 1-Acetoxypyren-3,6,8-trisulfonic acid, Rhodamine 123, Rhodamine 110, EOSIN, 7-Aminoactinomycin D, Sulforhodamine G, Rhodamine 6G Perchlorate, Evans Blue, Nile Red Phenoxazon 9, Rhodamine B, Pyronin B, Sulforhodamine B, 3.3-Dimethyloxadicarbocyanine, Sulforhodamine 101, Naphtho Fluorescein, Carboxynaphtho Fluorescein, Thionin, Nile Blue A Perchlorate, 3.3-Diehtylthiadicarbocyanine, Methyl-DOTCI and IR 144, and said indicator component comprises a material selected from the group consisting of Propyl Red, p-Nitrophenol, Azolitmin, Bromocresol Purple, Chlorophenol Red, 3.6-Dihydroxy xanthone, Alizarin, Bromxylenol Blue, 3.6-Dihydroxy phthalic dini, m-Dinitrobenzoyleneurea, Bromthymol Blue, Aurin (Aosolic acid), Phenol Red, Cleves Acid, Orcinaurine, Resolic acid and Neutral Red.

## Patentansprüche

1. Verfahren zur Feststellung biologischer Aktivitäten in einem Probengut, darin bestehend,
- daß eine Probe des zu untersuchenden Probenguts und eine Nährlösung in einen verscließbarn, optisch durchlässigen Behälter gefüllt werden, welcher zumindest einen aktivierbaren, inerten Fluorophor und zumindest eine Indikatorsubstanz enthält, die auf Änderungen der Konzentration zumindest eines der im Behälter vorliegenden Stoffe mit einer Änderung ihrer optischen Eigenschaften reagiert,
- daß der Behälter verschlossen wird,
- daß die Probe Bedingungen ausgesetzt wird, welche bei Vorliegen von Mikroorganismen in der Probe metabolische Prozesse ermöglichen, wobei die Konzentration zumindest eines Stoffes während dieser metabolische Prozesse vermindert wird,
- daß der inerte Fluorophor dazu angeregt wird, Fluoreszenzstrahlung abzugeben,
- daß die vom inerten Fluorophor abgegebene Fluoreszenzstrahlung mit den optischen Veränderungen des Indikators moduliert und so ein moduliertes Fluoreszenzsignal erzeugt wird, und ferner,
- daß der zeitliche Verlauf des Signals gemessen wird, wodurch Änderungen festgestellt werden können, die auf das vorhandensein von Mikroorganismen schließen lassen.

2. Verfahren nach Anspruch 1 , dadurch gekennzeichnet, daß im verschließbaren, optisch durchlässigen Behälter ein optischer Sensor vorgesehen ist, welcher aus einem inerten Fluorophor und einem CO₂-empfindlichen Farbindikator besteht, und daß das Fluoreszenzsignal des Fluorophors mit dem CO₂-empfindlichen Farbindikator in Abhängigkeit von der ansteigenden CO₂-Konzentration moduliert wird.

3. Verfahren nach Anspruch 1, dadurch gekennzeichnet, daß die intensität der vom inerten Fluorophor abgegebenen Fluoreszenzstrahlung mit der zumindest einen Indiktorsubstanz in Abhängigkeit von deren optischen Eigenschaften moduliert wird.

4. Verfahren nach Anspruch 1, dadurch gekennzeichnet, daß die Strahlung vor Anregung des inerten Fluorophors mit der zumindest einen Indiktorsubstanz in Abhängigkeit von deren optischen Eigenschaften moduliert wird.

5. Vorrichtung zur Feststellung biologischer Aktivitäten in einem Probengut, bestehend aus
- einem verschließbaren Behälter mit einem optisch durchlässigen Wandteil,
- einer Nährlösung und einer darin befindlichen Probe des zu untersuchenden Probenguts, die in diesem Behälter enthalten sind,
- Einrichtungen, die auf den Inhalt des Behälters einwirken und metabolische Prozesse ermöglichen, wobei sich die Konzentration zumindest eines der im Behälter vorliegenden Stoffe ändert,
- zumindest einem optischen Sensor, welcher am optisch durchlässigen Wandteil angebracht ist, bestehend aus einem aktivierbaren, inerten Fluorophor und einer Indiktorsubstanz, die auf von metabolischen Prozessen ausgelöste Änderungen der Konzentration zumindest eines der im Behälter vorliegenden Stoffe mit einer Änderung ihrer optischen Eigenschaften reagiert, dadurch gekennzeichnet, daß die vom inerten Fluorophor abgegebene Fluoreszenzstrahlung durch die optischen Änderungen der Indikatorsubstanz moduliert wird und ein moduliertes Fluoreszenzsignal erzeugt,
- einer Anregungseinrichtung zur Beleuchtung des zumindest einen optischen Sensors durch den optisch durchlässigen Wandteil des Behälters hindurch, sodaß der inerte Fluorophor des optischen Sensors zur Abgabe entsprechender Fluoreszenzsignale angeregt wird,
- einer Detektionseinrichtung, welche durch den optisch durchlässigen Wandteil des Behälters dem zumindest einen optischen Sensor zugeordnet ist, sodaß die Änderung der Fluoreszenzsignale erfaßt werden kann.

6. Vorrichtung nach Anspruch 5, dadurch gekennzeichnet, daß der optische Sensor geeignet ist, Konzentrationsänderungen eines Stoffes aus der Gruppe CO₂, O₂, H⁺(pH), NH₄ ⁺, H₂ und Metallionen anzugeben.

7. Vorrichtung nach Anspruch 5, dadurch gekennzeichnet, daß zumindest zwei optische Sensoren kombiniert werden, die einen Bisensor zur Messung von Änderungen der O₂- und pH-Konzentration bilden.

8. Vorrichtung nach Anspruch 5, dadurch gekennzeichnet, daß zumindest drei optische Sensoren kombiniert werden, die einen Triseiisor zur Messung von Änderungen der CO₂-, O₂- und pH-Konzentration bilden.

9. Vorrichtung nach Anspruch 5, dadurch gekennzeichnet, daß einer der optischen Sensoren ein CO₂-Sensor ist, bestehend aus
- einer CO₂-permeablen Siliconmatrix,
- einem in der Matrix suspendierten Fluoreszenzstoff, und zwar Carboxynaphthofluorescein, und
- einer Indikatorsubstanz in der Matrix, in Form einer wässerigen Bromthymolblau-Emulsion.

10. Vorrichtung nach Anspruch 5, dadurch gekennzeichnet, daß der inerte Fluorophor aus einem Stof der Gruppe 1-Acetoxypyren-3,6,8-trisulfonsäure und DCM 4-Dicyanomethylen-2-methyl-6 (p-dimethylaminostyrol) 4H-Pyran besteht, und daß die Indikatorsubstanz aus einem Stoff der Gruppe p-Nitrophenol, m-Dinitrobenzoylenharnstoff Azolitmin und Bromxylenolblau besteht.

11. Vorrichtung nach Anspruch 5, dadurch gekennzeichnet, daß der inerte Fluorophor aus einem Stoff der Gruppe Thionin, 3.3-Dimethyloxadicarbocyanin, Carboxynaphthofluorescein und Sulforhodamin 101 besteht, und daß die Indikatorsubstanz aus einem Stoff der Gruppe Cleve-Säure, Orcinaurin, p-Nitrophenol, 3,6-Dihydroxyxanthenon, Bromxylenolblau und Bromthymolblau besteht.

12. Vorrichtung nach Anspruch 5, dadurch gekennzeichnet, daß der inerte Fluorophor aus einem Stoff der Gruppe 3.3-Diäthylthiadicarbocyanin und Nilblau besteht, und daß die Indikatorsubstanz aus einem Stoff der Gruppe Cleve-Säure, Azolitmin, Bromkresolpurpur, Alizarin und Propylrot besteht.

13. Vorrichtung nach Anspruch 5, dadurch gekennzeichnet, daß der inerte Fluorophor aus einem Stof der Gruppe 1-Hydroxypyren-3,6,8-trisulfonsäure, 1-Acetoxypyren-3,6,8-trisulfonsäure, Rhodamin 123, Rhodamin 110, EOSIN, 7-Aminoactinomycin D, Sulforhodamin G, Rhodamin 6G Perchlorat, Evansblau, Nilrot Phenoxazon 9, Rhodamin B, Pyronin B, Sulforhodamin B, 3.3-Dimethyloxadicarboxyanin, Sulforhodamin 101, Naphthofluorescein, Carboxynaphthofluorescein, Thionin, Nilblau A Perchlorat, 3.3-Diäthylthiadicarbocyanin, Methyl-DOTCI und IR 144 besteht, und daß die Indikatorsubstanz aus einem Stoff der Gruppe Propylrot, p-Nitrophenol, Azolitmin, Bromkresolpurpur, Chlorphenolrot, 3.6-Dihydroxyxanthenon, Alizarin, Bromxylenolblau, 3.6-Dihydroxyphthalsäure dini, m-Dinitrobenzoylenharnstoff, Bromthymolblau, Aurin (Rosolsäure), Phenolrot, Cleve-Säure, Orcinaurin, Resolsäure und Neutralrot besteht.

## Revendications

1. Procédé de détection des activités biologiques dans un échantillon qui comprend:
- l'introduction d'un prélèvement de l'échantillon et d'un milieu de culture dans un récipient transparent scellable contenant au moins un fluorophore inerte, activable et au moins un composant indicateur qui modifie ses caractéristiques optiques en réponse à des modifications dans la concentration d'au moins une substance dans ce récipient, - le scellement du dit récipient,
- l'exposition de l'échantillon à des conditions qui rendent possible que des activités métaboliques aient lieu en présence de micro-organismes dans le prélèvement, dans lequel la concentration d'au moins une substance est modifiée pendant les activités métaboliques,
- l'excitation du dit fluorophore inerte de façon à émettre une radiation d'émission fluorescente,
- la modulation de la radiation d'émission fluorescente du fluorophore inerte avec les modifications optiques de l'indicateur, de façon à produire un signal fluorescent modulé et la surveillance du signal pendant une période, pour les modifications qui indiquent la présence de micro-organismes.

2. Procédé selon la revendication 1, caractérisé en ce que le dit récipient scellable, transparent est pourvu d'un détecteur optique qui inclut un fluorophore inerte et un indicateur de couleur sensible au CO₂ et caractérisé en ce que le signal fluorescent du fluorophore est modulé avec l'indicateur de couleur sensible au CO₂ en réponse à l'augmentation de la concentration en CO₂.

3. Procédé selon la revendication 1, caractérisé en ce que l'intensité de la radiation d'émission fluorescente émise par le fluorophore inerte est modulée par au moins un composant indicateur en réponse à ses caractéristiques optiques.

4. Procédé selon la revendication 1, caractérisé en ce que la radiation préalable à l'excitation du fluorophore inerte, est modulée par au moins un composant indicateur en réponse à ses caractéristiques optiques.

5. Appareil pour la détection des activités biologiques dans un échantillon qui inclut:
- un récipient scellable comprenant un segment de paroi transparent,
- un milieu de culture et un prélèvement de l'échantillon scellés à l'intérieur dans ledit récipient,
- des moyens pour agir sur les teneurs dudit récipient pour rendre possible des activités métaboliques qui modifient la concentration d'au moins une substance dans ledit récipient,
- au moins un détecteur optique attaché audit segment de paroi transparent, ledit détecteur comprend un fluorophore inerte activable et un composant indicateur qui modifie ses caractéristiques optiques en réponse aux modifications dans la concentration d'au moins une substance dans ledit récipient causées par des activités métaboliques,
caractérisé en ce que la radiation d'émission fluorescente du fluorophore inerte est modulée par les modifications optiques du composant indicateur de façon à produire un signal de fluorescence modulé.
- des moyens d'excitation pour illuminer au moins ledit détecteur optique à travers le segment de paroi transparent dudit récipient de façon à exciter ledit fluorophore inerte dudit détecteur optique pour émettre les signaux correspondants fluorescents,
- des moyens de détection couplés optiquement à travers ledit segment de paroi transparent dudit récipient pour au moins un détecteur optique de détection des changements des signaux fluorescents.

6. Appareil selon la revendication 5, caractérisé en ce que ledit détecteur optique est sensible pour indiquer des modifications dans la concentration d'une structure choisie dans le groupe qui consiste en CO₂, O₂, H⁺ (PH), NH₄ ⁺, H₂ et les ions métalliques.

7. Appareil selon la revendication 5, caractérisé en ce qu'au moins deux desdits détecteurs optiques sont combinés pour former un bidétecteur pour indiquer les modifications de concentration de O₂ et de pH.

8. Appareil selon la revendication 5, caractérisé en ce qu'au moins trois desdits détecteurs optiques sont combinés pour former un détecteur triple pour indiquer les modifications de concentration de CO₂, O₂ et du pH.

9. Appareil selon la revendication 5, caractérisé en ce qu'un desdits détecteurs optiques est un détecteur de CO₂ et comprend:
- une matrice de silicone perméable à CO₂,
- un matériau fluorescent à base de carboxynaphtofluorescéine mis en suspension dans une matrice et,
- une émulsion aqueuse de bleu de bromothymol en tant qu'indicateur dans cette matrice.

10. Appareil selon la revendication 5, caractérisé en ce que ledit fluorophore inerte comprend un matériau choisi dans le groupe qui consiste dans l'acide 1-acétoxypyrène,-3,6,8-trisulfonique, et DCM (4-dicyanométhylène-2-méthyl-6 (p-diméthylaminostyrène [4H]-pyranne, et ce composant indicateur comprend un produit choisi dans le groupe qui consiste en le p-nitrophénol, la m-dinitrobenzoylèneurée, l'azolithmine et le bleu de bromoxylénol .

11. Appareil selon la revendication 5, caractérisé en ce que ledit fluorophore inerte comprend un produit choisi dans le groupe qui consiste en la thionine, la 3,3-diméthyloxadicarbocyanine, la carboxynaphtofluorescéine et la sulforhodamine 101 et ledit composant indicateur comprend un produit choisi dans le groupe qui consiste en l'acide de Clèves, l'orcinaurine, le p-nitrophénol, la 3,6-dihydroxyxanthone, le bleu de bromoxylènol et le bleu de bromothymol.

12. Appareil selon la revendication 5, caractérisé en ce que ledit fluorophore inerte comprend un matériau choisi dans le groupe qui consiste en la 3,3-diéthylthiadicarbocyanine et le bleu Nil, et ledit composant indicateur comprend un produit choisi dans le groupe qui consiste en l'acide de Clèves, l'azolithmine, le pourpre de bromocresol, l'alizarine et le rouge propyle.

13. Appareil selon la revendication 5, caractérisé en ce que ledit flurophore inerte comprend un produit choisi dans le groupe qui consiste en l'acide 1-hydroxypyrène-3,6,8 trisulfonique, l'acide-1-acétoxypyrène-3,6,8-trisulfonique, la rhodamine 123, la rhodamine 110, l'éosine, la 7-aminoactinomycine D, la sulforhodamine G, la rhodamine 6G perchlorate, le bleu Evans, le rouge Nil Phénoxazone 9, la rhodamine B, la pyronine B, la sulforhodamine B, la 3,3-diméthyloxadicarbocyanine, la sulforhodamine 101, la naphtofluorescéine, la carboxynaphtofluorescéine, la thionine, le perchlorate de bleu Nil A, la 3,3-diéthylthiadicarbocyanine, le méthyl-DOTCI et le produit IR144 et ledit composant indicateur comprend un produit choisi dans le groupe qui consiste en le rouge propyle, le p-nitrophénol, l'Azolithmine, le pourpre de bromocresol, le rouge de chlorophénol, la 3,6-dihydroxyxanthone, l'alizarine, le bleu de bromoxylénol, 3,6-dihydroxyphtalique, la m-dinitrobenzoylène urée, le bleu de bromothymol, l'aurine (Acide aosolique), le rouge de phénol, l'acide de Clèves, l'orcinaurine, l'acide résolique et le rouge neutre.
